# EUROPÄISCHE PATENTANMELDUNG

(11) **EP 1 512 384 A1**
(43) Veröffentlichungstag der Anmeldung: **09.03.2005**
(21) Anmeldenummer: 03016441.2
(22) Anmeldetag: 21.07.2003
(51) Int. Cl.: A61F 2/44

(54) **Bandscheibenprothese**

(71) Anmelder: Cervitech, Inc., Rockaway, NJ 07866 (US)
(72) Erfinder: Keller, Arnold, 23863 Kayhude (DE)
(74) Vertreter: Glawe, Delfs, Moll & Partner

(57) **Zusammenfassung**

Bandscheibenprothese mit zwei Deckplatten und einem Prothesenkern, der mit einer der Deckplatten (1) durch komplementär hinterschnittene Verbindungsprofile (8, 8', 14, 14') verbunden ist. Die Verbindungsprofile (8, 8', 14, 14') umfassen mindestens ein Paar von zur AP-Richtung (10) symmetrisch und zueinander winklig angeordneten Profilabschnitten (9, 16).

## Beschreibung

Ein bekannter Typ von Bandscheibenprothesen (WO 01/01893) weist zwei Deckplatten aus Metall und einen Prothesenkern aus Polyethylen auf, der mit einer der beiden Deckplatten ein Gleitflächenpaar bildet und an der anderen Deckplatte befestigt ist. Für die Befestigung sind an der Deckplatte Randleisten vorgesehen, deren hinterschnittenes Verbindungsprofil zusammenwirkt mit einem komplementären Verbindungsprofil an der Unterseite des Prothesenkerns. Am rechten und linken lateralen Rand der Deckplatte und des Prothesenkerns sind solche Verbindungsprofile parallel zueinander vorgesehen. Außerdem befindet sich ein solches, geradlinig gestrecktes Profil am dorsalen Rand der Deckplatte und des Prothesenkerns. Am ventralen Rand fehlt ein solches Profil, so daß der Prothesenkern von der ventralen Seite her in die Verbindungsprofile der Deckplatte eingeschoben werden kann. Durch ein Paar von am ventralen Rand vorgesehenen Rastelementen wird der Prothesenkern in der montierten Stellung festgehalten. Obwohl die Verbindungsprofile nahezu spielfrei hergestellt werden, besteht ein wichtiges Anliegen der Prothesenherstellung darin, auch im Falle von Herstellungstoleranzen ein Bewegungsspiel des Prothesenkerns gegenüber der ihn haltenden Deckplatte auszuschließen. Insbesondere sollen Rotationsbewegungen des Prothesenkerns gegenüber der ihn haltenden Deckplatte ausgeschlossen werden. Die Erfindung hat erkannt, daß dies nicht nur durch genaue Vertigung, sondern auch durch eine besondere Anordnung der Verbindungsprofile verbessert werden kann.

Erfindungsgemäß ist vorgesehen, daß die Verbindungsprofile mindestens ein Paar von zur AP-Richtung symmetrisch und zueinander winklig angeordneten Verbindungsprofilabschnitten umfassen. Unter der AP-Richtung ist die antero-posteriore Richtung zu verstehen. Die erfindungsgemäß winklig zueinander angeordneten Profilabschnitte erzeugen eine Dreieckskonfiguration, die einer Verdrehung des Prothesenkerns gegenüber der Deckplatte entgegenwirkt, falls zwischen den Verbindungsprofilen der Deckplatte und des Prothesenkern Spiel vorhanden ist. Die Verbindungsprofile können im wesentlichen ausschließlich von derartigen winklig zueinander angeordneten Profilabschnitten gebildet sein. Diese können aber auch zusätzlich zu parallel an den lateralen Rändern der Deckplatte und des Prothesenkerns angeordneten Verbindungsprofilen vorgesehen sein. Sie sollen im wesentlichen geradlinig gestreckt sein und sind vorzugsweise zumindest dorsal angeordnet. Der Winkel zwischen den winklig zueinander angeordneten Profilabschnitten soll 150° nicht überschreiten. Vorzugsweise liegt die Grenze etwa bei 140°.

Die Erfindung wird im folgenden näher unter Bezugnahme auf die Zeichnung erläutert, die ein vorteilhaftes Ausführungsbeispiel veranschaulicht. Darin zeigen:
- Fig. 1: eine perspektivische Ansicht der Endplatte mit daran befestigtem Prothesenkern,
- Fig. 2 bis 4: unterschiedliche Ansichten derselben Endplatte ohne Prothesenkern und
- Fig. 5 und 6: perspektivische Ansichten des Prothesenkerns.

Die Endplatte 1, die normalerweise als untere Endplatte eingesetzt wird, hat einen etwa ovalen Umriß und weist eine gezahnte Befestigungsfläche 2 zur Auflage auf den Wirbelkörper und einen Flansch 3 auf, der an der ventralen Seite des Wirbelkörpers zu liegen kommt und durch Schraubenlöcher 4 damit verschraubt werden kann. Auf der der Befestigungsfläche 2 gegenüber liegenden Seite, die normalerweise oben liegt, bildet die Deckplatte 1 ein Lager für den Prothesenkern 5. Dieses Lager besteht aus einer ebenen Fläche 6 und einem diese umgebenden Rand 7, der aus zwei parallelen Abschnitten 7' an den lateralen Seiten der Platte und einem dorsalen Rand 7" zusammengesetzt ist. Die Außenkontur des Randes 7 entspricht der ovalen Kontur der Deckplatte. Der Rand ist auf seiner der Fläche 6 zugewendeten Seite mit einer Profilleiste 8 und darunter mit einem Hinterschnitt 8' versehen. Das dadurch an der Innenseite des Rands 7 gebildete Verbindungsprofil verläuft in den lateralen Abschnitten 7 und 7' gerade und auf beiden Seiten parallel. Im dorsalen Abschnitt 7" setzt sich das Verbindungsprofil aus zwei Abschnitten 9 zusammen. In jedem dieser Abschnitte verläuft das Verbindungsprofil geradlinig. Die Profile beider Abschnitte sind symmetrisch zur AP-Achse 10 des Implantats angeordnet und schließen ein Winkel α ein, der im dargestellten Beispiel bei 140° liegt.

Die Oberseite des Rands 7 bildet eine ebene Fläche 6', die parallel zur Lagerfläche 6 verläuft und gleichfalls eine Lagerfläche für den Prothesenkern bildet.

Der Flansch 3 der Deckplatte enthält einen Schlitz 11, der lotrecht zur Verbindungsprofil 6 verläuft und dessen Funktion später erläutert wird.

Der Prothesenkern 5 bildet auf seiner Oberseite 12 eine Gleitfläche, die mit der zweiten, nicht dargestellten Deckplatte zur Bildung eines Gelenks zusammenwirkt. Der Umriß des Prothesenkerns stimmt mit demjenigen der Deckplatte 1 überein. Auf ihrer Unterseite weist sie eine ebene Fläche 13 auf, die zur Auflage auf der Lagerfläche 6 der Endplatte 1 bestimmt ist und im wesentlichen dieselbe Umrißgestalt hat. Sie wird begrenzt durch ein Verbindungsprofil, das von einer Profilleiste 14 und einem Hinterschnitt 14' gebildet ist. Dieses Verbindungsprofil stimmt mit dem Verbindungsprofil 8, 9 der Randleiste 7 der Deckplatte 1 komplementär überein. Insbesondere setzt sich das Verbindungsprofil aus zwei lateralen Abschnitten 15 und zwei dorsalen Abschnitten 16 zusammen, die jeweils den lateralen und dorsalen Abschnitten des Rands 7 der Deckplatte 1 entsprechen und mit diesen formflüssig zusammenwirken können.

Außerhalb des Verbindungsprofils 14, 14' befindet sich an der Unterseite des Prothesenkerns 5 eine ebene Fläche 17, die der Lagerfläche 6' auf der Oberseite des Rands 7 der Deckplatte 1 entspricht und tragend mit ihr zusammenwirkt, wenn der Prothesenkern an der Deckplatte 1 montiert ist.

Die Montage findet dadurch statt, daß der Prothesenkern mit seinem Verbindungsprofil 14, 14' in die entsprechenden Verbindungsprofile 8, 8' von der offenen, ventralen Seite her eingeschoben wird. Wenn der Prothesenkern vollständig eingeschoben ist, greift seine Profilleiste 14 nicht nur im Bereich der seitlichen Abschnitte 7', sondern auch des dorsalen Abschnitts 7" in den Hinterschnitt 8' des Rands 7 der Deckplatte ein, wobei die Verbindungsprofile im wesentlichen vollflächig aneinander liegen.

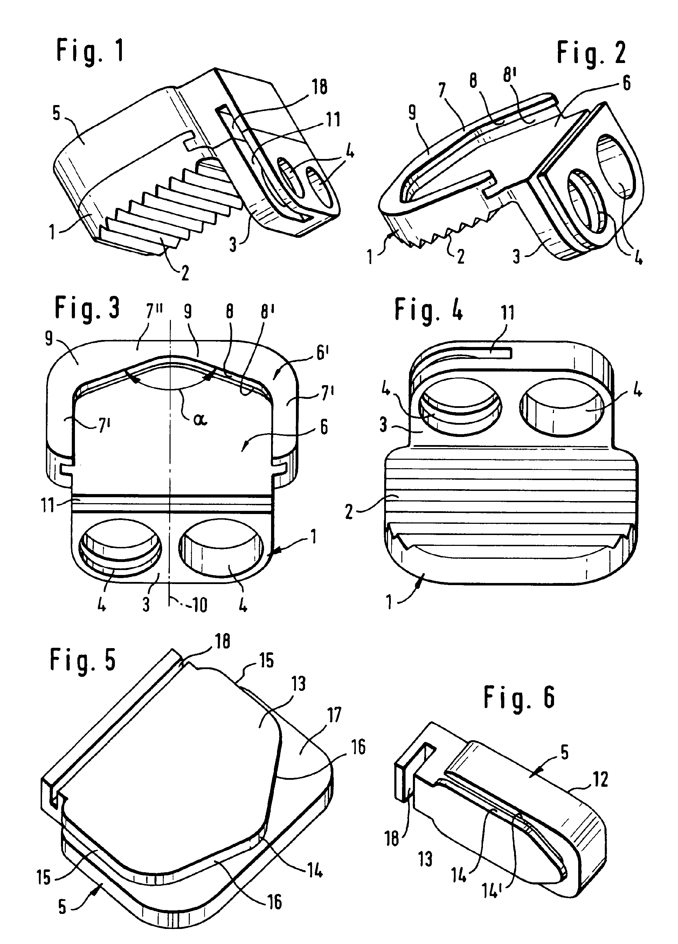

An derjenigen Stelle, an der sich der Schlitz 11 im Spalt 3 befindet, weist der Prothesenkern eine Nut 18 auf, die mit dem Spalt 11 fluchtet, wenn der Prothesenkern seine Endstellung erreicht hat, in welcher die dorsalen Verbindungsprofile aneinander anschlagen. Es wird eine nicht gezeigte Lamelle in den Spalt 11 und die Nut 18 eingeschoben, die den Prothesenkern in dieser Position gegenüber der Deckplatte 1 sichert. Die Lamelle ihrerseits wird dadurch gesichert, daß sie an der Stelle des in Fig. 3 links erscheinenden Schraubenlochs ebenfalls eine Bohrung aufweist, durch die eine etwaige Befestigungsschraube hindurchgeht.

Dadurch, daß die Verbindungsprofile des Prothesenkerns und der Deckplatte mit den sich zu einem Dreieck ergänzenden Abschnitten 9 bzw. 16 ausgestattet sind, wird im Vergleich mit bekannten Prothesen eine gesteigerte Lagesicherheit des Prothesenkerns gegenüber der Deckplatte 1 erzielt. Insbesondere wird seine Sicherheit gegenüber Relativbewegungen im Rotationssinne dadurch gesteigert.

## Patentansprüche

1. Bandscheibenprothese mit zwei Deckplatten und einem Prothesenkern (5), der mit einer der Deckplatten (1) durch komplementär hinterschnittene Verbindungsprofile (8, 8', 14, 14')verbunden ist, **dadurch gekennzeichnet, daß** die Verbindungsprofile (8, 8', 14, 14') mindestens ein Paar von zur AP-Richtung (10) symmetrisch und zueinander winklig angeordneten Profilabschnitten (9, 16) umfassen.

2. Bandscheibenprothese nach Anspruch 1, **dadurch gekennzeichnet, daß** die zueinander winklig angeordneten Profilabschnitte (9, 16) im wesentlichen geradlinig gestreckt sind..

3. Bandscheibenprothese nach Anspruch 1 oder 2, **dadurch gekennzeichnet, daß** die zueinander winklig angeordneten Profilabschnitte (9, 16) dorsal angeordnet und durch eine ventral angeordnete Einrichtung (11, 18) in Eingriff gehalten sind.

4. Bandscheibenprothese nach Anspruch 3, **dadurch gekennzeichnet, daß** die dorsal angeordneten Profilabschnitte (9, 16) zusätzlich zu einem Paar lateral angeordneter Profilabschnitte (7') vorgesehen sind.

5. Bandscheibenprothese nach Anspruch 1 oder 2, **dadurch gekennzeichnet, daß** der Winkel (α) zwischen den zueinander winklig angeordneten Profilabschnitten (9, 16) nicht größer als 150° ist.
